# EUROPEAN PATENT APPLICATION

(11) **EP 1 604 669 A1**
(43) Date of publication of application: **14.12.2005**
(21) Application number: 04291458.0
(22) Date of filing: 10.06.2004
(51) Int. Cl.: A61K 31/663, A61P 9/00, G01N 33/15

(54) **Use of ATP analogues for treatment of cardiovascular diseases**

(71) Applicant: INSTITUT NATIONAL DE LA SANTE ET DE LA RECHERCHE MEDICALE (INSERM), 75654 Paris Cédex 13 (FR)
(72) Inventor: Martinez, Laurent, 81000 ALBI (FR); Terce, Francois, 31140 LAUNAGUET (FR); Collet, Xavier, 31000 TOULOUSE (FR); Barbaras, Ronald, 31700 BEAUZELLE (FR); Boeynaems, Jean-Marie, 1780 WEMMEL (BE)
(74) Representative: Le Coupanec, Pascale A.M.P.

(57) **Abstract**

Use of *N*-alkyl-2-substituted ATP analogues, pharmaceutically acceptable salts, metabolites or prodrugs thereof, for the preparation of a medicament intended to be used in the prevention and/or the treatment of cardiovascular diseases, pharmaceutical compositions comprising such compounds and a method for screening such compounds.

## Description

The present invention relates to the use of specific *N*-alkyl-2-substituted ATP analogues for the manufacturing of a medicament intended to be used in the treatment and/or the prevention of cardiovascular diseases associated with an excess of cholesterol.

Cholesterol is a soft, waxy substance present in all parts of the body including the nervous system, muscles, liver, intestine and heart. Cholesterol is mainly provided to the organism either by consumption of foods of animal origin or from the liver metabolism, and is used for the production of hormones, bile acid, and vitamin D.

Blood circulating cholesterol is present under the form of macromolecular complexes of lipids and proteins that are classified by density and electrophoretic mobility. Such macromolecular complexes are termed chylomicrons, Very Low-Density Lipoprotein (VLDL), VLDL-remnants, Intermediate-Density Lipoprotein, Low-Density Lipoprotein (LDL) and High-Density Lipoprotein (HDL).

Chylomicrons, VLDL and LDL are mainly involved in the transport of cholesterol from intestine and liver towards peripheral tissues. LDL-cholesterol is commonly known as "bad cholesterol". In return, the HDL particles are involved in the transport of cholesterol from the peripheral tissues, such as fibroblasts and macrophages, towards the liver, and are commonly known as "good cholesterol".

Atherosclerosis is a physiopathological process, the development of which relies upon numerous risk factors, among which the most critical is an excess of cholesterol, and in particular an accumulation of cholesterol at peripheral tissues level. Atherosclerosis disease represents the first cause of death in industrialized countries (Miller *et al.,* Lancet, 1977, 965-968). Total cardiovascular death from cardiovascular diseases related to atherosclerosis are expected to almost double from 13.1 millions in 1990 to 24.8 millions in 2020 (Poulter, Heart, 2003, 89 (suppl. 2): ii2-ii5).

Atherosclerosis is a disease of large and medium-sized muscular arteries and is characterised by endothelial dysfunction, vascular inflammation, and the build-up of lipids, cholesterol, calcium and cellular debris within the intima of the vessel wall.

The mechanisms of atherogenesis remain uncertain, but probable causes include injury of endothelial cells by oxidized LDL-cholesterol. Circulating monocytes infiltrate the intima of injuried vessel walls, and these tissue macrophages act as scavenger cells, taking up LDL cholesterol and forming the characteristic foam cells of early atherosclerosis.

Progression of the diseases results in a formation of a fibrous plaque which induces vascular remodelling, progressive luminal narrowing, blood-flow abnormalities, and compromised oxygen supplied to the target organ.

Cardiovascular diseases such as stable angina pectoris, intermittent claudication and mesenteric angina are examples of the clinical consequences of the mismatch between inadequate blood supply to an organ, in the event of increase of the metabolic activity, and oxygen demand.

Rupture of a plaque or denudation of the endothelium overlying a fibrous plaque may result in exposure of the highly thrombogenic sub-endothelium and lipid core. These exposures may result in thrombus formation which may partially or completely occlude flow in the involved artery. Instable angina pectoris, myocardial infarction, ischemic attack and stroke are examples of the clinical sequelae of partial or complete acute occlusion of an artery.

Hyperlipidemia is an established risk factor for atherosclerosis and convincing evidence that lowering serum cholesterol reduces the risk of subsequent coronary heart diseases events and overall mortality exist.

Current strategies used for lowering cholesterol at peripheral tissues level mainly involve action on LDL-cholesterol particles. This may be done at dietary level and includes restriction of caloric intake, saturated fats, cholesterol and increase physical activity.

Epidemiologic data have allowed to negatively correlate plasma level of HDL to incidence of cardiovascular diseases (Gordon *et al.,* circulation, 1989, 79: 8-15; Miller *et al.,* Am. J Cardiol. 1990, 65: 1-5). HDL particles are currently the only known physiological factor being protective in regard of myocardial infarction (Frick *et al.,* Drugs, 1990, 40 Suppl 1: 7, 12). This is usually attributed to their property to purify cells from their excess of cholesterol, and bring it back to the liver, thus reducing the extent of atherosclerosis lesions. This "cholesterol reverse transport" is the main way of cholesterol elimination. In this process, HDL particles mediate the efflux and the transport of cholesterol from peripheral cells to the liver for final catabolism and bile secretion. Recently, it has been identified a cell surface ATP synthase as a high-affinity receptor for HDL apolipoprotein A-I (apoA-I) on human hepatocytes. Stimulation of this ectopic form of ATP synthase by apoA-I triggered a low affinity receptor-dependent HDL endocytosis by a mechanism strictly related to the generation of ADP (Martinez *et al.,* 2003, Nature, 421:75-79).

Other means to reduce accumulation of cholesterol in peripheral tissues rely upon the use of pharmacological tools such as antilipemic agents, as for example nicotinic acid derivatives or fibrates compounds, hydroxymethylglutaryl-Coenzyme A reductase inhibitors, as for example statins, or peroxisome proliferators-activated receptor-γ activators, as for example thiazolidinediones. Those agents mainly act by reducing LDL-cholesterol plasma level, and slightly increasing HDL-cholesterol plasma level.

However, none of those treatments are fully satisfactory, and there is still an unmet need for the prevention and/or the treatment of cardiovascular diseases resulting from an excess of cholesterol, and in particular from the accumulation of cholesterol in the peripheral tissues.

Unexpectedly, the inventors have discovered that the use of a *N*-alkyl-2-substituted ATP derivative resulted in an increased of endocytosis of HDL-cholesterol at the liver level, and consequently increased the cholesterol reverse transport, and decreased the accumulation of cholesterol at peripheral tissues level.

According to one of its advantages, the present invention allows for increasing the flow of cholesterol out of peripheral tissues.

According to another of its advantages, the present invention allows for reducing and/or preventing the occurring of an excess of cholesterol, and in particular accumulation of cholesterol at peripheral tissues level.

According to another of its advantages, the present invention allows for the prevention and/or the reducing of generation of atheromatous plaques.

According to another of its advantages, the present invention allows for the prevention and/or the reducing of thrombus generation.

According to another of its advantages, the present invention allows for the prevention and/or the reducing of cardiovascular diseases generation.

According to another of its advantages, the present invention allows for identifying new compounds allowing to improve cholesterol reverse transport.

More particularly, the present invention relates to the use of a compound of formula (I): 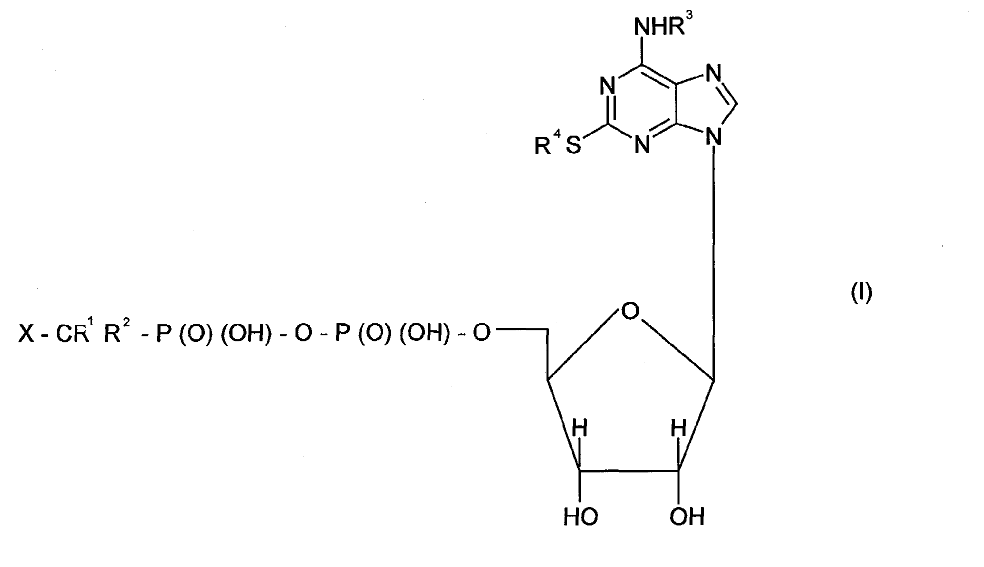 wherein:
- R¹ and R² independently represent hydrogen or halogen,
- R³ and R⁴ independently represent phenyl, or alkyl C₁₋₆ optionally substituted by one or more substituents selected from OR⁵, alkylthio C₁₋₆, NR⁶R⁷, phenyl, COOR⁸ and halogen, with R⁵, R⁶, R⁷ and R⁸ independently representing hydrogen or alkyl C₁₋₆, and
- X represents an acidic moiety, and pharmaceutically acceptable salts, metabolites or prodrugs thereof, for the preparation of a medicament intended to be used in the prevention and/or treatment of a cardiovascular disease associated with cholesterol accumulation in peripheral tissues.

According to another aspect, the present invention relates to the use of a compound of formula (I), as above-defined, for the preparation of a medicament intended to be used in the prevention and/or the treatment of a cardiovascular disease associated with an impaired cholesterol reverse transport.

According to another of its aspect, an object of the present invention is related to the use of a compound of formula (I), as above-defined, for the preparation of a medicament intended to be used in the prevention and/or the treatment of a cardiovascular disease associated with an impaired cholesterol hepatic endocytosis.

According to another aspect, the present invention relates to a method of prevention and/or treatment of a cardiovascular disease associated with accumulation of cholesterol in peripheral tissues, comprising the administering to an animal in need thereof of a therapeutically effective amount of a compound of formula (I), as above-defined.

According to another aspect, the present invention relates to a method of prevention and/or treatment of a cardiovascular disease associated with an impaired reverse cholesterol transport comprising the administering to an animal in need thereof of a therapeutically effective amount of a compound of formula (I), as above-defined.

According to another aspect, the present invention relates to a method of prevention and/or treatment of a cardiovascular disease associated with an impaired hepatic endocytosis of HDL cholesterol comprising the administering to an animal in need thereof a therapeutically effective amount of a compound of formula (I), as above-defined.

According to another aspect, the present invention relates to a pharmaceutical composition comprising a compound of formula (I), as above-defined, and a second compound allowing the increase of HDL-cholesterol and/or decrease of LDL-cholesterol plasma level.

According to another aspect, the present invention relates to a method for screening a compound modulating HDL cholesterol internalization comprising at least a step of exposing a sample of cells expressing a P2Y₁₃ receptor to a compound to be tested under conditions favourable for internalization of said HDL-cholesterol by said sample of cells and a step of detecting an optional internalization.

For the purpose of the present invention,
- the expressions "internalization" or "endocytosis" are used interchangeably, and are understood to mean a physiological process by means of which, biological molecules are captured and ingested by cells,
- the expression "excess of cholesterol" is understood to mean that the plasma level of a human individual of LDL-cholesterol is varying from 100 to 129 mg/dL, in particular from 130-159 mg/dL, in particular from 160-189 mg/dL, and more particularly is above 190 mg/dL, or otherwise that the plasma level of total cholesterol of a human individual is varying from about 200 mg/dL to about 239 mg/dL and in particular is above 240 mg/dL.

In relation to cardiovascular disease, the term "prevention" refers to preventing cardiovascular disease from occurring, i.e. a *N*-2-alkyl-substituted ATP analogue is administered prior to the onset of the cardiovascular disease condition. This means that the compounds of the present invention can be used as prophylactic agents to impede a cardiovascular disease.

In relation to atherosclerosis, the term "prevention" refers to preventing atherosclerosis from occurring, i.e. a *N*-2-alkyl-substituted ATP analogue is administered prior to the onset of the atherosclerosis condition. This means that the compounds of the present invention can be used as prophylactic agents to impede atherosclerosis lesions generation.

The *N*-alkyl-2 substituted ATP analogues to be used according to the present invention are in particular described in the European patent EP 0 683 789 and in the publication of Ingall *et al.,* (J. Med. Chem., 1999, 42:213-220), which are incorporated herein by reference.

In particularly, *N*-alkyl-2 substituted ATP analogues to be used in the present invention may be represented by the following formula (I): 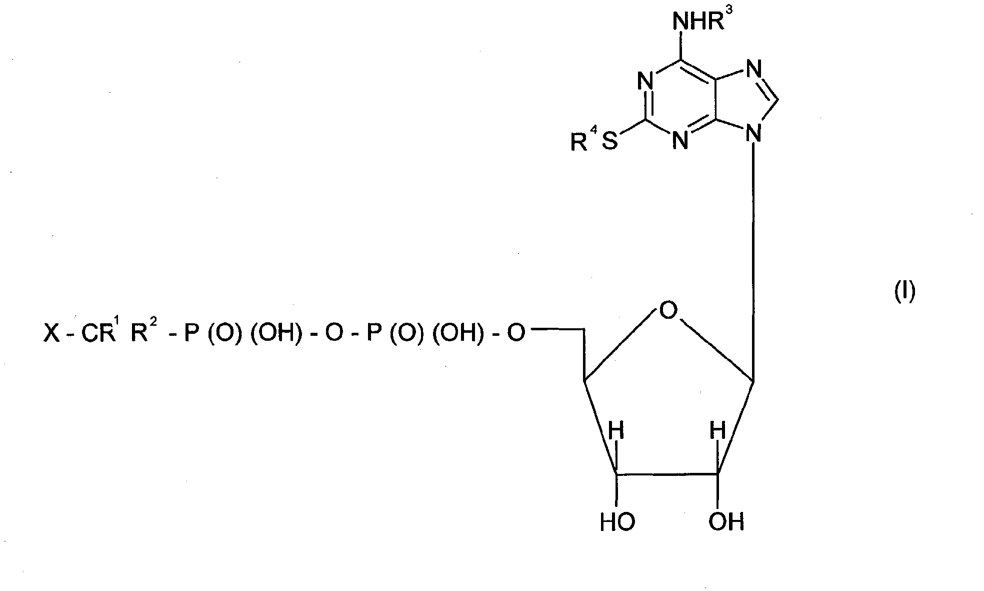 wherein
i) R¹ and R² independently are a halogen chosen among Cl, F, Br and I,
ii) R³ and R⁴ independently represent phenyl or alkyl C₁₋₆ optionally substituted by one or more substituents selected from OR⁵, alkylthio C₁₋₆, NR⁶R⁷, phenyl, COOR⁸ and halogen, with R⁵, R⁶, R⁷ and R⁸ independently being hydrogen or alkyl C₁₋₆, and
iii) X represents a phosphoric moiety, pharmaceutically acceptable salts, metabolites or prodrugs thereof

For the purpose of the present invention, the expression "prodrug" is dedicated to refer to any substance that gives arise to a pharmaceutically active form of a compound of formula (I), although inactive itself.

In the scope of the present invention, the term "metabolite" is considered to be any substance resulting from the metabolism of a compound of formula (I), and which is active as for the intended use according to the present invention.

In the purpose of the present invention, the terms "derivative" and "analogue" are used interchangeably, and are understood to mean inorganic or organic salts, esters or amides, in particular ammonium salts, of compounds of formula (I). More generally, its salts include not only the addition salts with carboxylic organic acids, like the acetate for example, but also other addition salts such as for example the trifluoroacetate, as well as the addition salts with inorganic acids such as the sulphate, hydrochloride and the like. The derivatives also include the salts resulting from the salivation of the carboxyl group and in particular the salts of alkali metals or alkaline earth metals such as the salts of sodium or of calcium, and in particular sodium salts.

According to a particular embodiment of the present invention, R¹ and R² are different or identical, and in particular are identical and more particularly are chlorine (Cl).

According to a particular embodiment of the present invention, R³ is a radical chosen among ethyl, butyl, methylethyl, methoxyethyl, methylthioethyl, trifluoroethyl, methoxycarbonylmethyl, dimethylaminoethyl, cyclopentyl, and phenyl, and in particular is methylthioethyl.

According to another particular embodiment of the present invention, R⁴ is a radical chosen among propyl, trifluoropropyl and cyclohexyl, and in particular is trifluoropropyl.

In a particular embodiment, the compounds of formula (I) may have the additional feature of presenting an affinity for the purinergic receptor P2Y₁₃, and in particular the human P2Y₁₃ receptor. This affinity may range from pM to mM and in particular from nM to µM values. This affinity results in an interaction between compounds of formula (I) and P2Y₁₃ receptor, translating itself in a biological response.

In a particular embodiment of the present invention, the compound of formula (I) is *N*-[2-(methylthio)ethyl]-2[(3,3,3-trifluoropropyl)thio]-5'-adenylic acid, monoanhydride with dichloromethylenebisphosphonic acid, salts, derivatives or prodrugs thereof, which is also known as AR-C69931MX.

It has been observed that the AR-C69931MX compound, which was initially described as an antagonist of P2Y₁₃ receptors (Marteau *et al.,* Mol. Pharmacol., 2003, 64:104-112) was, unexpectedly, able to increase over 30% the HDL-cholesterol endocytosis by HepG₂ cells.

Similar results were obtained with this compound in a model of *in situ* perfused mouse liver, wherein the application of AR-C69931MX was able to stimulate the internalization of TG-HDL₂ and cholesteryl-ether-TG-HDL₂ by 34% and 27%, respectively.

Due to its effect on HDL internalization, these compounds and more generally compounds as defined in formula (I) are thus useful for improving and/or increasing the cholesterol reverse transport process.

Therefore, the *N*-alkyl-2 susbtituted ATP analogues, and in particular the ARC69931MX, reveal themselves particularly useful for the manufacturing of a medicament intended to be used in the prevention and/or the treatment of a cardiovascular disease associated with an impaired cholesterol reverse transport and/or impaired cholesterol hepatic endocytosis, and/or with accumulation of cholesterol at peripheral tissues level.

The main consequence of an excess of cholesterol, and in particular of an accumulation of cholesterol at peripheral tissues level, is the formation of atherosclerosis plaques, also known as atherogenesis. The development and accumulation of atherosclerosis plaques results in numerous cardiovascular diseases which are for example coronary artery disease, coronary heart disease, stable or unstable angina pectoris, stroke, transient ischemic attack, intermittent claudication, mesenteric angina, and myocardial infarction.

Accordingly, the *N*-alkyl-2 substituted ATP derivatives, and in particular the AR-C69931MX compound, are particularly useful for the preparation of a medicament intended to be used in the prevention and/or the treatment of a cardiovascular disease as above-defined.

According to a particular embodiment of the present invention, the medicament manufactured with at least one *N*-alkyl-2 ATP substituted analogues, and in particular AR-C69931MX, are intended to be administered to an animal in need thereof.

For the purpose of the present invention, the term "animals" includes mammals such as human and non-human animals, such as farm (agricultural) animals, especially the animals of economic importance such as gallinaceous birds, bovine, ovine, caprine and porcine mammals, especially those that produce products suitable for the human consumption, such as meat, eggs and milk. Further, the term is intended to include fish and shellfish, such as salmon, cod, Tilapia, clams and oysters. The term also includes domestic animals such as dogs and cats. The term is also used to refer to laboratory animals which include, but are not limited to, rodents such as mice, rats, guinea-pigs or hamsters.

In accordance with the methods indicated above, preferred embodiments are as follows: said animal is a human, an agricultural animal, a laboratory animal and/or a domestic or pet animal.

In a particular embodiment of the present invention, the *N*-alkyl-2-substituted ATP analogues, and in particular the AR-C69931MX compound, are used in an animal in need thereof at a therapeutically effective amount, that is to say at the minimal amount necessary to observe the expected effect, and in particular for preventing and/or treating a cardiovascular disease resulting from an excess of cholesterol, in particular from an accumulation of cholesterol at peripheral tissues level.

The therapeutically effective amount may be determined by the skilled person by any conventional method known in the art.

According to a particular embodiment, this therapeutically effective amount may vary from about 0.1 mg/kg/day to about 1000 mg/kg/day, in particular from about 0.1 mg/kg/day to about 100 mg/kg/day, and more particularly from about 0.1 mg/kg/day to about 50 mg/kg/day, and in particular from about 0.1 mg/kg/day to about 10 mg/kg/day.

A pharmaceutical composition, intended to be used for the prevention and/or the treatment of a cardiovascular disease resulting from excess of cholesterol, and in particular from an accumulation of cholesterol in peripheral tissues, should include in particular less than 80% by weight, more particularly less than 50% by weight, and in particular from 0.1 to 20% by weight relative to the total weight of the composition, of a compound of formula (I), or a pharmaceutically acceptable salt, or a metabolite or a prodrug thereof as above-defined, in a mixture with a pharmaceutically acceptable diluant or carrier.

Such a medicament, or pharmaceutical composition may be provided under a suitable galenic form for parenteral administration, or administration *per os.* Parenteral administration includes, for example, intravenous or intraarterial administration, subcutaneous administration, inhalation, rectal administration, dermal or intradermal administration. Suitable galenic form for parenteral administration are for example liquid forms, suppositories, patch, or transdermic device. Suitable galenic form for administration *per os* are for example tablets, capsules and dragees.

Owing to its effect on cholesterol reverse transport, and in particular on the increase of cholesterol hepatic endocytosis, it may be particularly advantageous to combine a compound of formula (I) with a second compound that allows the increase of plasma level of HDL-cholesterol and/or the decrease of plasma level of LDL-cholesterol.

Accordingly, according to a particular embodiment, the present invention is directed to a pharmaceutical composition comprising, in combination, a compound of formula (I), as above defined, and a second compound allowing the increase of plasma level of HDL-cholesterol and/or the decrease of plasma level of LDL-cholesterol, for use in a therapy.

Among the compounds known to decrease plasma level of LDL, and possibly increase HDL plasma level, mention may be made of statins, fibrates, thiazolidinediones and nicotinic acid derivatives (Rifkind, Tex Heart Inst. J. 1990, 17(4):346-352).

Statins belongs to the family of hydroxymethylglutaryl-Coenzym A reductase inhibitors (HMG-CoA reductase inhibitors) which inhibits an enzyme that catalyses the rate-limiting step in cholesterol biosynthesis, resulting in upregulation of LDL-receptors in response to the decrease in intra-cellular cholesterol. Compounds that belong to the statins family are for example pravastatin, simvastatin, lovastatin, fluvastatin, atorvastatin and rosuvastatin. Those compounds are usually used at a dose ranging from 10 to 80 mg/day.

Of compounds belonging to the fibrates family, mention may be made of fenofibrates, bezafibrate and gemfibrosil. Those compounds are, respectively, usually used at a dose of about 160 mg/day and about 1200 mg/day. The precise mechanism of fibrates is complex and incompletely understood. They increase the activity of lipoprotein lipases and enhance the catabolism of triglycerides-rich lipoproteins, which is responsible for an increase in the HDL-cholesterol fraction.

Among nicotinic acid derivatives, mention may be made of niacin which inhibits the hepatic secretion of VLDL-cholesterol. Niacin has been demonstrated to lower LDL-cholesterol and triglyceride levels, respectively, and to raise the HDL-cholesterol level. The compound is usually used at a dose ranging from 2 to 6 g/day.

Regarding thiazolidinediones family, mention may be made of rosiglitazone, which act through peroxysome proliferators-activated receptor-γ activation, enhancing expression of the adenosine triphosphate-binding cassette protein A1 (ABCA1) and therefore promoting cholesterol efflux and reverse cholesterol transport. This compound is usually used at a dose of about 8 mg/day.

The appropriate dose of those above-defined compounds may be determined by the skilled person according to the considered compound and the pathological status of the animal to which the compound is to be administered.

Owing to the additive and/or synergic effect obtained, when administrated with a compound of formula (I), the second compound which may increase plasma level of HDL-cholesterol and/or decrease plasma level of LDL-cholesterol, as well as the compound of formula (I), may be used at a dose lower than the dose usually recommended. Such lower dose may be easily determined by the skilled person according to any known conventional method.

Such method may be for example, comparing the effect on atherosclerosis lesions and/or cardiovascular disease status, of the simultaneous or separate administration to an animal in need thereof, of both compounds with the effect of the administration of each compound alone.

The compound of formula (I) and the second compound, as above-defined, may be presented in the same container, separately into two distinct formulations, or simultaneously into the same formulation. When presented into two distinct formulations, those compounds may be administered simultaneously or separately in the time.

For the purpose of the present invention, the term "formulation" is understood to mean a composition comprising one or more active ingredient, such as a compound of formula (I), and one or more appropriate pharmaceutically acceptable excipient or diluent or carrier and intended to be presented in an appropriate galenic form.

Therefore, according to a particular embodiment, the present invention relates to a pharmaceutical composition comprising at least a compound of formula (I) and at least a second compound, as above-defined, presented separately or simultaneously in a same container.

According to a further aspect, an object of the present invention relates to a method for preventing and/or treating a condition where excess of cholesterol, and in particular accumulation of cholesterol in peripheral tissues, is involved which comprises administering a therapeutically effective amount of a compound of formula (I), as above-defined, to an animal in need thereof.

In a particular embodiment, the method according to the present invention comprises the administration, in combination with a compound of formula (I) as above-defined, of a second compound that allows to increase the plasma level of HDL-cholesterol and/or the decrease of plasma level of LDL-cholesterol.

The striking observation by the inventors that the P2Y₁₃ receptors activation by a compound of formula (I), in contrast to the P2Y₁ and P2Y₆ receptors, resulted in an increase of HDL-cholesterol internalization at the level of hepatic cells, allowed the development of a new method of screening for identifying new compounds increasing HDL-cholestereol hepatic endocytosis, and consequently cholesterol reverse transport.

Therefore, the present invention according to another of its aspects, relates to a method for screening a compound modulating HDL-cholesterol internalization, comprising at least a step of exposing a sample of cells expressing P2Y₁₃ receptors to a compound to be tested, under conditions favourable for internalization of HDL-cholesterol by said sample of cells and a step of detecting optional internalization.

According to a particular embodiment, of the present invention, the method of screening comprises the steps of:
a) incubating a first sample of said cells in the presence of said compound and HDL-cholesterol and a second sample of said cells in the absence of said compound and in presence of HDL-cholesterol, both said samples being under conditions which permit binding of said compound to P2Y₁₃ receptor and internalization of HDL-cholesterol,
b) detecting internalization of HDL-cholesterol in said first and second samples, and
c) comparing internalization of HDL-cholesterol into said first and second samples.

For the purpose of the present invention, the terms "conditions favourable for the internalization of the HDL-cholesterol", are understood to mean, that, in addition to the P2Y₁₃, the cells of the sample also express sites of affinity or receptors able to bind HDL-cholesterol, and that upon the binding of the compound to be screened to the P2Y₁₃ receptors, there may be, or not, an increase or a stimulation of the internalization of said sites of affinity or receptors by said cells.

For the purpose of the present invention, the expressions "sites of affinity or receptors able to bind HDL-cholesterol" are understood to mean a molecule on the cell surface that has an affinity for cholesterol, cholesterolesters, phospholipids, triglycerides and/or proteins from the macromolecular complex HDL-particle.

According to a particular embodiment, the cells to be used in the method, as above-defined, may be chosen among hepatic cell lines, hepatic cells of primary cultures, hepatic cells of explanted liver tissues, hepatic cells of *in vitro* isolated liver and hepatic cells of *in vivo* liver. More particularly, the cells to be used in the method according to the present invention, are hepatic cell lines, such as for example HepG₂ or HuH7 cells.

According to a particular embodiment, the hepatic cells of *in vivo* liver, may be from a perfused animal liver model wherein the liver is perfused in situ with, for example, HDL-cholesterol and, the compound to be screened.

In particular, a suitable animal for said perfused liver model may be chosen from mouse, rat, guinea-pig, cat, dog or pig, and is in particular a mouse.

In regard of the present invention a suitable animal which may be used in the above-defined model, is for example a mouse knocked-out in regard of P2Y₁, and particularly P2Y₁^{-/-} C57/BL/6J mouse.

According to a particular embodiment, the HDL-cholesterol which may be used in the method according to the present invention, may be chosen from triglycerides-rich HDL2 (TG-HDL2) cholesteryl-ether labeled triglycerides-rich HDL2 (cholesteryl-ether-TG-HDL2), esters of HDL-cholesterol, HDL3, cholesteryl-ether-labeled HDL3, and potentially all HDL lipoparticles.

The HDL-cholesterol to be used in the method may be radio-labelled or fluorescent-labelled cholesterol. Among the radio-labelled HDL-cholesterol which may be used in the method according to the present invention, mention may be made of ¹²⁵I-TG-HDL2, ³H-cholesteryl-ether-TG-HDL₂, ³H-cholesteryl-ether-TG-HDL3 or ¹²⁵I-HDL₃.

Among the fluorescent-labelled-cholesterol which may be used in the method according to the present invention mention may be made of BODIPY-Cholesterol.

The measure of HDL-cholesterol internalization into cells may be carried out by measuring amount of sites of affinity or receptors binding HDL-cholesterol inside those cells, after applying compounds to be screened. To follow the internalization of these receptors, these latters may be tagged by means of specific radio-labelled or fluorescent-labelled antibodies. Radio-element which may be used to label such antibodies may be chosen from ³H or ¹²⁵I. The antibodies may also be labelled with fluorescent dyes chosen, for example, among BODIPY or fluorescein-5-isothiocyanate.

The obtaining of antibodies directed to a particular protein and the labelling of such antibodies, with radio-element or fluorescent dyes, are of general knowledge of the skilled person.

According to a particular embodiment, the internalization of the receptor binding HDL-cholesterol, may be measured by recording the variation of fluorescence of a dye, the fluorescence of which varies according to the pH of its environment. Such dye may be, for example, green-fluorescent-protein (GFP) which may be linked to the receptor binding HDL-cholesterol by any known molecular biology techniques from the skilled person or may be a fluorescent dye labelling an antibody used to tag the said receptor.

According to another embodiment, the site of affinity or receptor binding HDL-cholesterol, the internalization of which is to be followed, may be in particular scavenger receptor class B type I (SR-BI), scavenger receptor class B type II (SR-BII) or human equivalent CD36-Limp 1 analogous 1 receptor (CLA-1).

According to another embodiment, the amount of HDL-cholesterol internalized may be measured by measuring the level of cholesterol or HDL-cholesterol into the cells after extraction.

HDL-cholesterol may be measured, for example, with an automatic analyser (Olympus AU400®) with commercial kits (Olympus Diagnostica GmbH, references OSR6187).

The invention will be more fully understood by reference to the following examples. They should not, however, be construed as limiting the scope of the invention.

### LEGENDS OF THE FIGURES

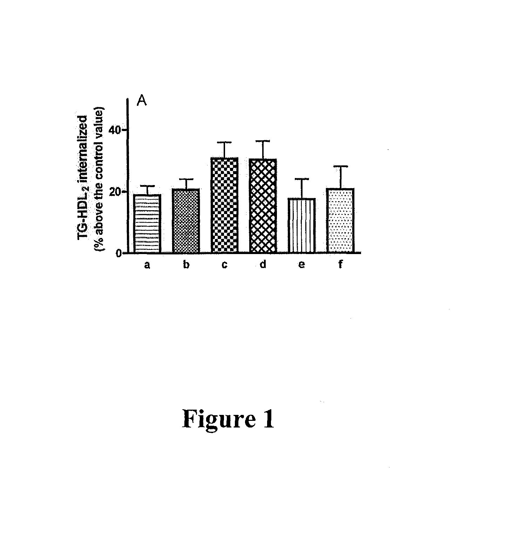

Figure 1: It shows effect of adenosine diphosphate (ADP) and pharmacological modulators of P2Y receptors on triglyceride-HDL₂ (TG-HDL₂) internalization by HepG₂ cells. Cells were incubated for 10 minutes at 37°C with 75 µg/ml¹²⁵I-TG-HDL₂ without (control) or with apolipoprotein A-1 10 µg/ml (a), ADP 100 nM (b), AR-C69931MX 100 nM (c), ADP 100 nM +AR-C69931MX 100 nM (d), ADP 100 nM + A2P5P 100 µM (e), ADP 100 nM + MRS2179 10 µM (f). Data are expressed as a percentage of internalized radioactivity with respect to the control value (set as zero) (n>5).

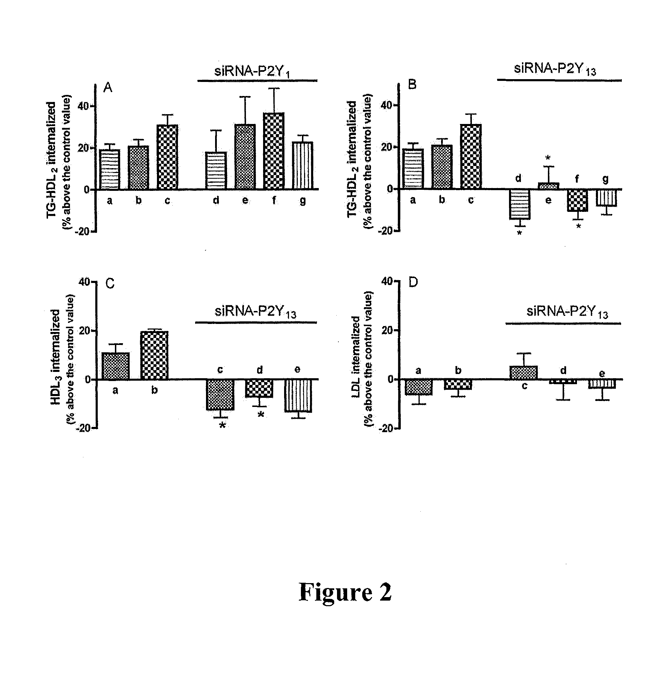

Figures 2: They show involvement of human P2Y₁ and P2Y₁₃ receptors in the internalization of lipoproteins by HepG₂ cells.
Figure 2A: It shows the involvement of human P2Y₁ receptor on TG-HDL₂ internalization. HepG₂ cells were transiently transfected with siRNA designated to human P2Y₁ receptor (columns d, e, f, g) or with non specific siRNA as control (a, b, c). 48 hours later, cells were incubated for 10 minutes at 37°C with 75 µg/ml ¹²⁵I-TG HDL₂ without (control) or with apolipoprotein A-1 10 µg/ml (a and d), ADP 100 nM (b and e), ARC69931MX 100 nM (c and f), PBS (g). Data are expressed as a percentage of internalized radioactivity with respect to the control value (set as 0) (n>5); (*) p<0.05 (unpaired t-test).
Figure 2B: It shows the involvement of P2Y₁₃ receptor on TG-HDL₂ internalization. Experiment was performed as in Figure 2A with non-specific control siRNA (a, b, c) or with siRNA directed to human P2Y₁₃ receptor (d, e, f, g) (n>7); (*) p<0.05 (unpaired t-test).
Figure 2C: It shows the involvement of P2Y₁₃ receptor on HDL₃ internalization. Cells were transiently transfected with siRNA designated to human P2Y₁₃ receptor (column c, d, e). 48 hours later, cells were incubated for 10 minutes at 37°C with 75 µg/ml ¹²⁵I-HDL₃ without (control) or with ADP 100 nM (a and c), AR-C69931MX 100 nM (b and d), PBS (e) (n>5); (*) p<0.05 (unpaired t-test).
Figure 2D: It shows the involvement of P2Y₁₃ receptor on LDL internalization. Experiment was performed as in Figure 2C except that HepG2 cells were incubated for 10 minutes at 37°C with 75 µg/ml ¹²⁵I-LDL (n>6).

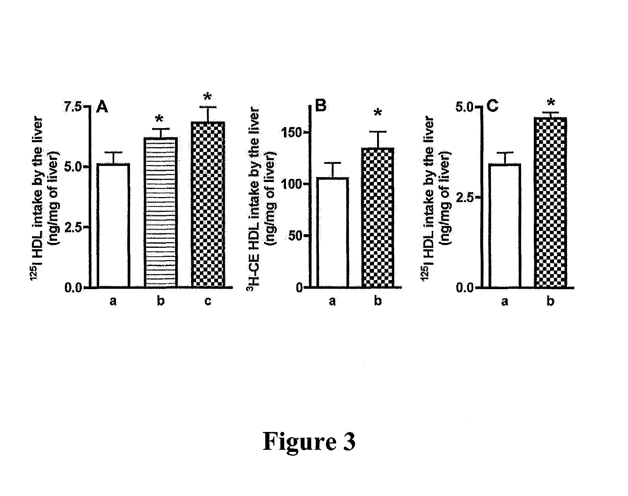

Figures 3: They show *in situ* TG-HDL2 endocytosis by perfused mouse liver.
Figure 3A: The liver from three groups of C57BL/6J male mice (8 weeks old) was perfused *in situ* for 10 minutes at 37°C in HBSS medium with 75 µg/ml of ¹²⁵I-TG-HDL₂ in the presence of PBS (a), apoA-I 10 µg/ml (b) or AR-C69931MX 10 µM (c). ¹²⁵I radioactivity associated with the livers was determined, (n>4); (*) (p<0.05) (unpaired t-test).
Figure 3B: The liver from C57BL/6J male mice (8 weeks old) was perfused *in situ* for 10 minutes at 37°C in HBSS medium with 75 µg/ml of ³H-cholesteryl-ether-TG-HDL₂ in the presence of PBS (a) or AR-C69931MX 10 µM (b). ³H radioactivity associated with the livers was determined (n>4); (*) (p<0.05) (unpaired t-test).
Figure 3C: The liver from P2Y₁^{-/-} C57BL/6J male mice (25 weeks old) was perfused *in situ* for 10 minutes at 37°C in HBSS medium with 75 µg/ml of ¹²⁵I-TG-HDL₂ in the presence of PBS (a) or AR-C69931MX 10 µM (b). ¹²⁵I radioactivity associated with the livers was determined (n>3); (*) (p<0.05) (unpaired t-test).

### Material and methods

ADP, A2P5P, MRS2179 were from SIGMA. [³H]-cholesteryl-oleylether (250 µCi TRK888) was from Amersham. AR-C69931MX used in the experiments was synthezised as already published (Ingall *et al.,* J. Med. Chem., 1999, 42:213-20).

Internalization assays were performed as described in Guendouzi *et al.,* (Biochemistry, 1998, 37: 14974-80) on HepG₂ cells, obtained from the American Type Culture Collection (HBM-8065). Cells were incubated for 10 minutes at 37°C with 75 µg/ml of either ¹²⁵I-TG-HDL₂, ¹²⁵I-HDL₃ or ¹²⁵I-LDL. After washing and dissociation at 4°C in phosphate buffered saline (PBS), cell radioactivity was determined using a Wallac 1261 multigamma apparatus.

Prior experiments, cells were plated in polylysine-coated 24-multiwell plates (Falcon) at 4 x 104 cells per well. Cells were grown in Dulbecco's modified Eagles medium supplemented with 10% fetal calf serum, 100 units/ml penicillin, and 100 µg/ml streptomycin at 37°C in a 5% CO2 and 95% air incubator. Medium was changed every 2 or 3 days, and the cells were subcultured every 7-8 days.

The silencing of the different P2Y receptors were carried-out using the siRNA technique. The siRNA sequenced targeting human P2Y₁₃ receptor (Gene Bank GB/EMBL/DDBJ accession number AF295368) was from position 406 or 359 relative to the start codon. The siRNA duplex (21-nt-RNAs) in deprotected and desalted form, the siRNA targeting human P2Y₁ receptor (a mix of 4 siRNAs, M-005689), and the non-specific control siRNA (D-001206-01) were purchased from Dharmacon Inc. Subconfluent HepG₂ cells were transiently transfected with siRNAs (final concentration, 100 nM) using Oligofectamine™ according to the manufacturer's protocole (Invitrogen). Cells were usually assayed 48 hours after transfection. Specific silencing effect was confirmed by at least three independent experiments.

Results are expressed as a percentage of internalization with respect to the control value (set at 0). Control corresponded to a basal value of internalization, amounting to 400 ng, 300 ng, 200 ng of TG-HDL₂, HDL₃ and LDL per mg of cells protein respectively for 10 minutes. Data as a mean ± SEM of at least 7, 5, 6 independent experiments, for TG-HDL₂, HDL₃ and LDL respectively.

The perfused mouse liver experiments were carried out on C57BL/6J male mice, 8 weeks old, (Charles River Cie) or P2Y₁^{-/-} mouse, 25 weeks old. Mouse were anesthetized by intraperitoneal injections of 200 µl ketamine hydrochloride (Merial)-xylazine hydrochloride 2% (Bayer)-PBS (1:1:3 v/v). Portal vein and superior cave vein were then canulated and the liver extensively washed 15 min at 1 ml/min, 37 °C with Hank's balanced salt solution (HBSS - Invitrogen). The livers were perfused *in situ* for 10 minutes at 37°C in HBSS medium containing 75 µg/ml of ¹²⁵I-TG-HDL₂ (5000 cpm/ng of proteins) or ³H-cholesteryl-ether-TG-HDL₂ (15000 dpm/mg of cholesteryl ester), with PBS apolipoprotein A-1 (ApoA-1,10 µg/ml) or AR-C69931MX (10 µM).

Livers were extensively washed at 4°C in PBS and radioactivity associated per org of liver was counted, (n=7 per group). Values represent means ± SEM, with p<0.05 (unpaired t-test).

### Example I : Effect of ADP and pharmacological modulators of P2Y receptors on TG-HDL₂ internalization by HepG₂ cells.

To measure modulations in HDL endocytosis, HDL₂ enriched in triglycerides (TG-HDL₂) were used as a substrate for internalization experiments (Guendouzi *et al.,* 1998, Biochemistry, 37:14974-80). This typical "post-prandial" HDL particle is able to bind and to be internalized only through low-affinity HDL binding sites on human hepatocytes, thus avoiding the contribution of the high affinity apoA-1 receptor, ATP-synthase (Martinez *et al.,* 2003, Nature, 421:75-9). In a pharmacological approach, A2P5P and MRS2179, specific antagonists of P2Y₁ receptors (Ralevic & Burnstock, 1998, Pharmacol. Rev., 50:413-92) and AR-C69931MX, a dual antagonist of P2Y₁₂ and P2Y₁₃ receptors were used (Ingall *et al.,* 1998, J. Med. Chem., 42:213-20; Marteau *et al.,* 2003, Mol. Pharmacol 64:104-12).

Free apoA-I, which generates ADP through the activation of the ectopic form of ATP synthase, and ADP (Fig. 1, see (a) and (b)) stimulated the internalization of ¹²⁵I-labelled -TG-HDL₂ by HepG₂ cells (20% above control cells).

Addition of the P2Y₁ receptor antagonists, A2P5P and MRS2179 (Fig. 1, see (e) and (f)) had no effect on the stimulation by ADP of the TG-HDL₂ internalization. Conversely, AR-C69931MX increased the ADP-dependent TG-HDL₂ internalization (over 30% above control cells, Fig. 1, see (d)), and interestingly, the same stimulation level was observed for AR-C69931MX in the absence of ADP (Fig. 1, see (c)), suggesting a common signalization pathway for both compounds as agonists of P2Y₁₃ receptors.

### Example II : Involvement of human P2Y₁ and P2Y₁₃ receptors in the internalization of lipoproteins by HepG₂ cells.

The specific implication of P2Y₁₃ receptors in the TG-HDL₂ endocytosis by HepG₂ cells was analyzed using small interference RNAs (siRNA) (Caplen & Mousses, 2003, Ann. N.Y. Acad. Sci. 1002:56-62) as tools to turn off P2Y receptor protein production.

It was firstly observed that P2Y₁ receptors silencing by a pool of 4 specific P2Y₁ receptors siRNAs, has no effect on the stimulation of the TG-HDL₂ endocytosis by apoA-I (Fig. 2A, see (a) and (d)), ADP (Fig. 2A, see (b) and (e)) or AR-C69931MX (Fig. 2A, see (c) and (f)).

By contrast, P2Y₁₃ receptor silencing by two different specific siRNAs showed a dramatic inhibition of the basal (unstimulated) TG-HDL₂ endocytosis (Fig. 2B, see (g)) as well as after stimulation by apoA-I (Fig. 2B; see (a) and (d)), ADP (Fig. 2B, see (b) and (e)) or AR-C69931MX (Fig. 2B, see (c) and (f)). A similar inhibition of internalization by silencing of P2Y₁₃ receptors was observed when using ¹²⁵I-labelled HDL₃, a major subclass of HDL (Fig. 2C). Indeed, P2Y₁₃ receptors siRNAs induced again a strong inhibition of basal HDL₃ internalization (Fig. 2C, see (e)) as well as after stimulation by ADP (Fig. 2C, see (a) and (c)) and AR-C69931MX (Fig. 2C, see (b) and (d)). Finally, the specificity of this endocytotic pathway towards HDL particles was confirmed by the lack of stimulation of internalization of ¹²⁵I-labelled LDL by ADP (Fig. 2D, see (a) and (c)) or AR-C69931MX (Fig. 2D, see (b) and (d)), both without or with P2Y₁₃ receptors silencing.

### Exemple III: In situ TG-HDL₂ endocytosis by perfused mouse liver

To estimate the physiological relevance of P2Y₁₃ receptor as partner in HDL endocytosis, ¹²⁵I-labelled-TG-HDL₂ internalization experiments on perfused mouse liver where carried out *in situ.*

Remarkably, apoA-I and AR-C69931MX (Fig. 3A, see (b) and (c)) induced a rapid (10 min) and marked increase (up to 34% over control) of TG-HDL₂ internalization by the liver, indicating that, at least in the mouse, P2Y₁₃ seems to be implicated in hepatic HDL endocytosis. Moreover, stimulated endocytosis was not restricted to the protein moiety of HDL (labelled with ¹²⁵I), because AR-C69931MX also stimulated the internalization of ³H-cholesteryl-ether-labelled TG-HDL₂ (up to 27% over control, Fig. 3B, see (b)).

Interestingly, the ratio of cholesteryl ester to protein internalized by the liver was up to 20 folds more important than in the original TG-HDL₂ solution, indicating a "selective uptake" of cholesteryl-ester in the mouse liver. This suggests that the scavenger receptor class B type I (SR-BI), a widely described HDL receptor (Acton *et al.,* 1996, Science, 271, 518-20), involved in HDL cholesterol uptake, might be implicated in the foregoing observations in the mouse.

Finally, as for human hepatocytes, HDL endocytosis in mouse liver was independent of P2Y₁ receptors as demonstrated by the marked increase (38% above the control) of TG-HDL₂ internalization by the liver of P2Y₁^{-/-} (Leon *et al.,* 1999, J. Clin. Invest., 104:1731-7), incubated with AR-C69931MX (Fig. 3C, see (b)).

In conclusion, *in vitro* and *in situ* observations demonstrate that P2Y₁₃ receptors, unlike P2Y₁ receptors, are major partners in the HDL endocytosis by hepatocytes.

## Claims

1. Use of a compound of general formula (I) : 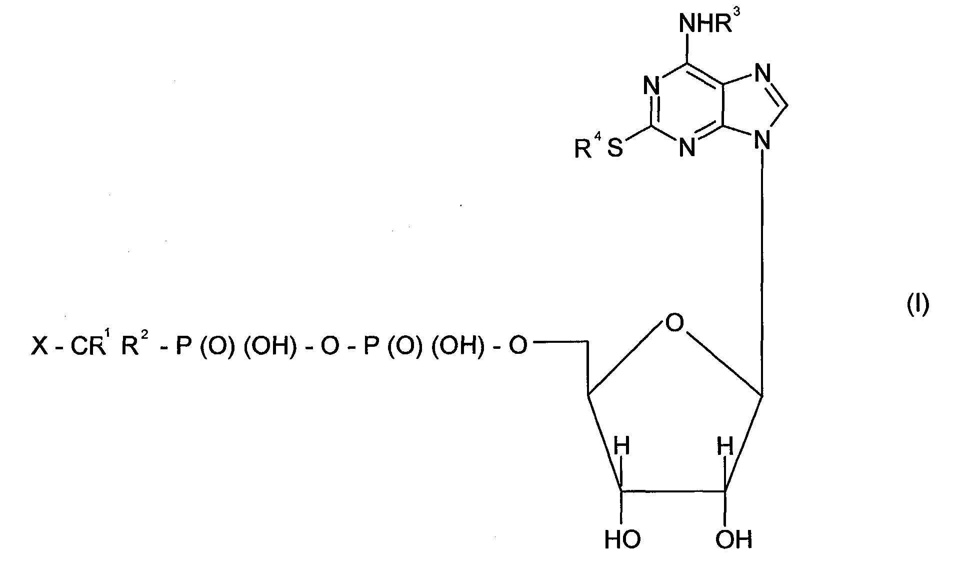 wherein
i) R¹ and R² independently represent hydrogen or halogen,
ii) R³ and R⁴ independently represent phenyl or alkyl C₁₋₆ optionally substituted by one or more substituents selected from OR⁵, alkylthio C₁₋₆, NR⁶R⁷, phenyl, COOR⁸ and halogen, with R⁵, R⁶, R⁷ and R⁸ independently being hydrogen or alkyl C₁₋₆, and
iii) X represents an acidic moiety,
pharmaceutically acceptable salts, metabolites or prodrugs thereof,
for the preparation of a medicament intended to be used in the prevention and/or the treatment of a cardiovascular disease associated with cholesterol accumulation in peripheral tissues, in an animal in need thereof.

2. Use of a compound of formula (I) : 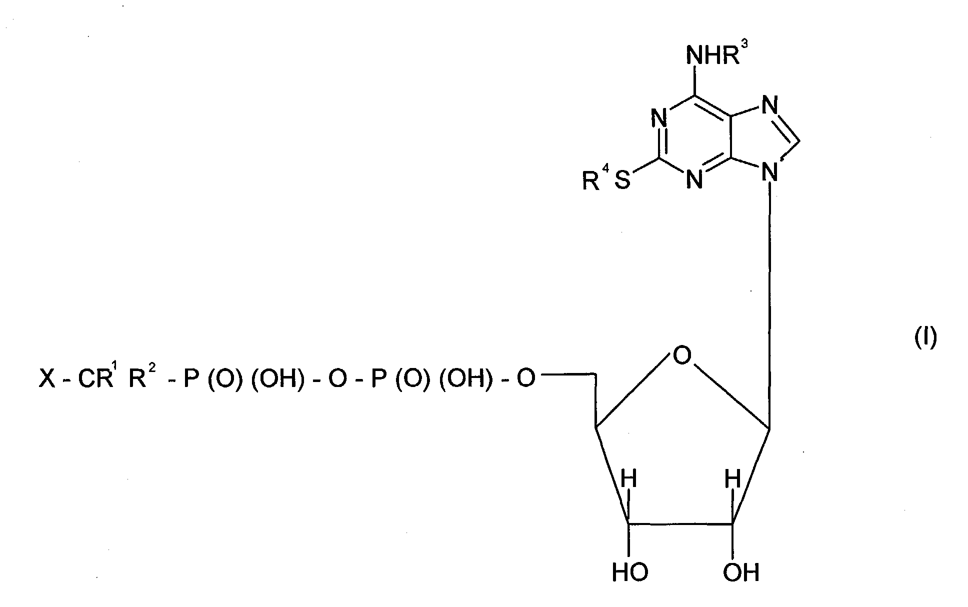 wherein
i) R¹ and R² independently represent hydrogen or halogen,
ii) R³ and R⁴ independently represent phenyl, or alkyl C₁₋₆ optionally substituted by one or more substituents selected from OR⁵, alkylthio C₁₋₆, NR⁶R⁷, phenyl, COOR⁸ and halogen, with R⁵, R⁶, R⁷ and R⁸ independently represent hydrogen or alkyl C₁₋₆, and
iii) X represents an acidic moiety,
pharmaceutically acceptable salts, metabolites or prodrugs thereof,
for the preparation of a medicament intended to be used in the prevention and/or the treatment of a cardiovascular disease associated with an impaired cholesterol reverse transport, in an animal in need thereof.

3. Use of a compound of formula (I) : 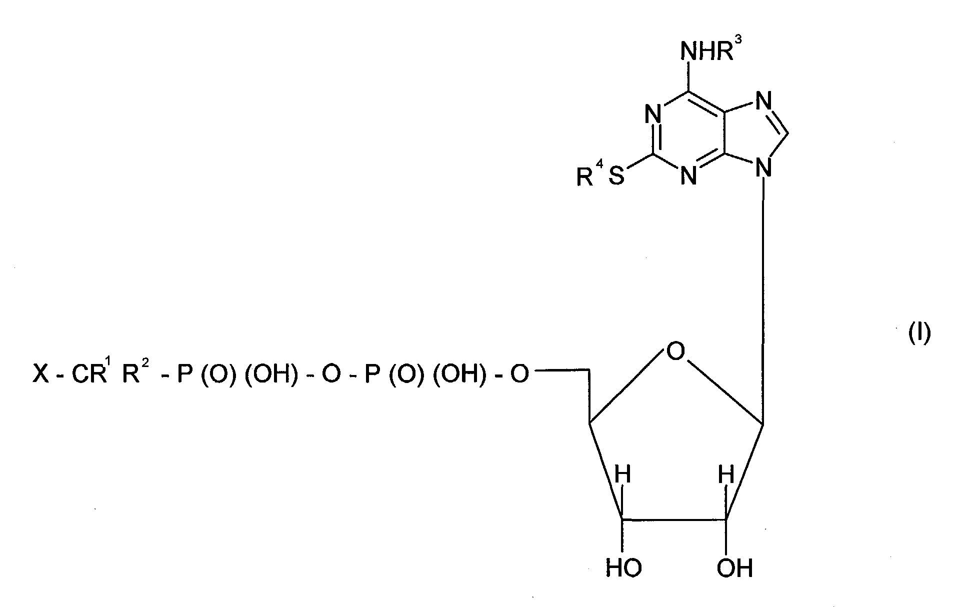 wherein
i) R¹ and R² independently represent hydrogen or halogen,
ii) R³ and R⁴ independently represent phenyl, or alkyl C₁₋₆ optionally substituted by one or more substituents selected from OR⁵, alkylthio C₁₋₆, NR⁶R⁷, phenyl, COOR⁸ and halogen, with R⁵, R⁶, R⁷ and R⁸ independently represent hydrogen or alkyl C₁₋₆, and
iii) X represents an acidic moiety,
pharmaceutically acceptable salts, metabolites or prodrugs thereof,
for the preparation of a medicament intended to be used in the prevention and/or the treatment of a cardiovascular disease associated with an impaired cholesterol hepatic endocytosis, in an animal in need thereof.

4. The use according to anyone of claims 1 to 3, wherein said cardiovascular disease is atherosclerosis, intermittent claudication, myocardial infarction, coronary artery disease, coronary heart disease, stable or unstable angina pectoris, stroke, and/or transient ischemic attack.

5. The use according to any one of claims 1 to 4, wherein R¹ and R² are an halogen, identical or different, chosen among Cl, F, Br and I, and in particular are identical, and more particularly are Cl.

6. The use according to any one of claims 1 to 5, wherein R³ is chosen among ethyl, butyl, methylethyl, methoxyethyl, methylthioethyl, trifluoroethyl, methoxycarbonylmethyl, dimethylaminoethyl, cyclopentyl, and phenyl, and in particular is methylthioethyl.

7. The use according to any one of claims 1 to 6, wherein R⁴ is chosen among propyl, trifluoropropyl and cyclohexyl, and in particular is trifluoropropyl.

8. The use according to any one of claims 1 to 7, wherein X is a phosphoric acid moiety.

9. The use according to any one of claims 1 to 8, wherein the compound of formula (I) is *N*-[2-(methylthio)ethyl]-2-[(3,3,3-trifluoropropyl)thio]-5'-adenylic acid, monoanhydride with dichloromethylenebisphosphonic acid, a pharmaceutically acceptable salt, a metabolite or a prodrug thereof.

10. The use according to any one of claims 1 to 9, wherein said compound of formula (I) is used at a therapeutically effective amount from about 0.1 mg/kg/day to about 1000 mg/kg/day, in particular from about 0.1 mg/kg/day to about 100 mg/kg/day, more particularly from about 0.1 mg/kg/day to about 50 mg/kg/day, and in particular from about 0.1 mg/kg/day to about 10 mg/kg/day.

11. The use according to any one of claims 1 to 10, wherein said compound of formula (I) is used in combination with a second compound allowing the increase of plasma level of HDL-cholesterol and/or the decrease of plasma level of LDL-cholesterol.

12. The use according to claim 11, wherein said second compound is chosen among statins, fibrates, thiazolidinediones and nicotinic acid derivatives.

13. The use according to any one of claims 1 to 12, wherein said animal is a human or a non-human mammal in need thereof.

14. The use according to claim 13, wherein said non-human mammal is chosen among domestic animals, laboratory animals or agricultural animals.

15. A pharmaceutical composition comprising, in combination, a compound of formula (I) as defined in any one of claims 1 to 3, 5 to 9, and a second compound allowing the increase of plasma level of HDL-cholesterol and/or decrease of plasma level of LDL-cholesterol.

16. The pharmaceutical composition according to claim 15, wherein said second compound is chosen among statins, fibrates, thiazolidinediones and nicotinic acid derivatives.

17. The pharmaceutical composition according to claim 15 or 16, wherein said compound of formula (I) and said second compound are presented separately or simultaneously in a same container.

18. The pharmaceutical composition according to any one of claims 15 to 17, for use in the prevention and/or the treatment of cardiovascular diseases said cardiovascular diseases are cardiovascular diseases associated with accumulation of cholesterol in peripheral tissues, cardiovascular diseases associated with an impaired cholesterol reverse transport, or cardiovascular diseases associated with an impaired cholesterol hepatic endocytosis.

19. Method for screening a compound modulating HDL-cholesterol internalization comprising at least a step of exposing a sample of cells expressing a P2Y₁₃ receptor to a compound to be tested under conditions favourable for internalization of said HDL-cholesterol by said sample of cells and a step of detecting optional internalization.

20. A method according to claim 19 said method comprising the steps of:
a) incubating a first sample of said cells in the presence of said compound and HDL-cholesterol and a second sample of said cells in the absence of said compound and in presence of HDL-cholesterol, both said samples being under conditions which permit binding of said compound to P2Y₁₃ receptor and internalization of HDL-cholesterol,
b) detecting internalization of HDL-cholesterol in said first and second sample and,
c) comparing internalization of HDL-cholesterol into said first and second samples.

21. The method according to claim 20, wherein said HDL-cholesterol is chosen from triglycerides-rich HDL₂, cholesteryl-ether-labeled triglycerides-riche HDL₂, esters of HDL-cholesterol, HDL₃ and cholesteryl-ether-labeled HDL₃.

22. The method according to claims 20 or 21, wherein said first and second samples of cells are chosen among hepatic cell lines, hepatic cell of primary cultures, hepatic cells of explanted liver tissues, hepatic cells of *in vitro* isolated liver and hepatic cells of *in vivo* liver, and in particular is HepG₂ or HuH7 cell line.

23. The method according to any one of claims 19 to 22, wherein said cells of said sample of cells express a receptor binding HDL-cholesterol, said receptor is radio-labelled or fluorescent-labelled, using green-fluorescent protein (GFP) labelling, and in particular is chosen from SR-BI, SR-BII and CLA-1 receptor.

## Amended claims

### Amended claims in accordance with Rule 86(2) EPC.

**1.** Use of a compound of general formula (I) : 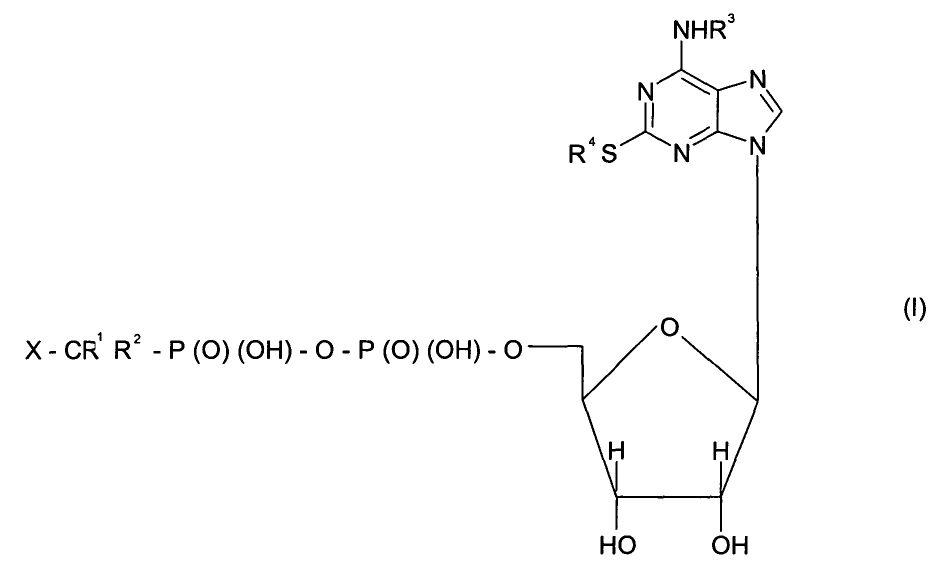 wherein
i) R¹ and R² independently represent hydrogen or halogen,
ii) R³ and R⁴ independently represent phenyl or alkyl C₁₋₆ optionally substituted by one or more substituents selected from OR⁵, alkylthio C₁₋₆, NR⁶R⁷, phenyl, COOR⁸ and halogen, with R⁵, R⁶, R⁷ and R⁸ independently being hydrogen or alkyl C₁₋₆, and
iii) X represents an acidic moiety,
pharmaceutically acceptable salts thereof,
for the preparation of a medicament intended to be used in the prevention and/or the treatment of atherosclerosis, in an animal in need thereof.

**2.** Use of a compound of formula (I) : 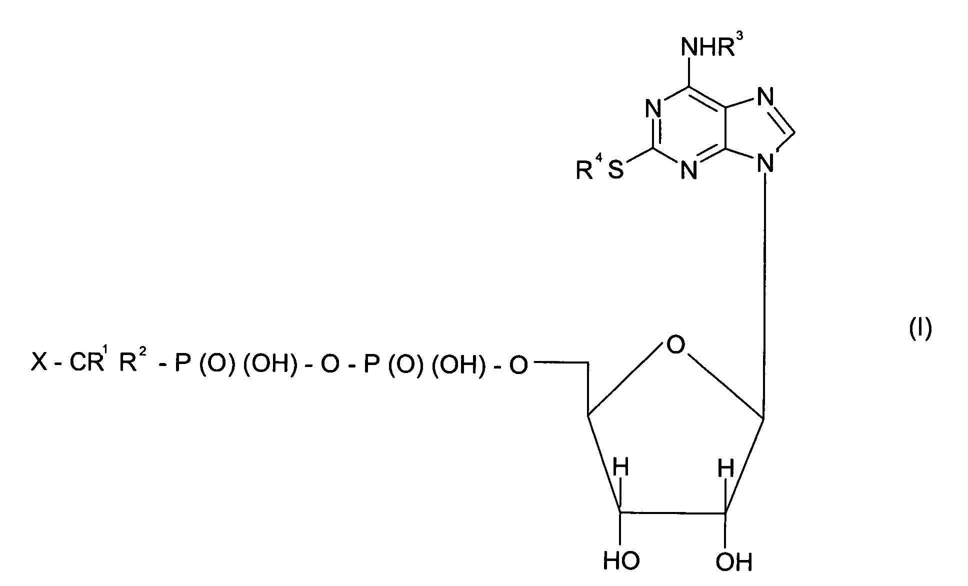 wherein
i) R¹ and R² independently represent hydrogen or halogen,
ii) R³ and R⁴ independently represent phenyl, or alkyl C₁₋₆ optionally substituted by one or more substituents selected from OR⁵, alkylthio C₁₋₆, NR⁶R⁷, phenyl, COOR⁸ and halogen, with R⁵, R⁶, R⁷ and R⁸ independently represent hydrogen or alkyl C₁₋₆, and
iii) X represents an acidic moiety,
pharmaceutically acceptable salts thereof,
for the preparation of a medicament intended to be used in the prevention and/or the reduction of generation of atheromatous plaques, in an animal in need thereof.

**3.** Use of a compound of formula (I) : 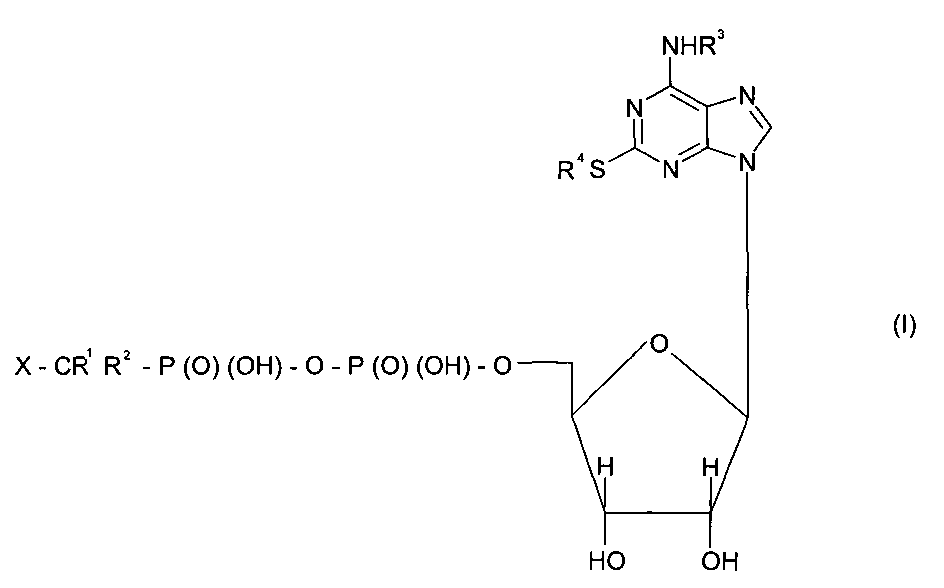 wherein
i) R¹ and R² independently represent hydrogen or halogen,
ii) R³ and R⁴ independently represent phenyl, or alkyl C₁₋₆ optionally substituted by one or more substituents selected from OR⁵, alkylthio C₁₋₆, NR⁶R⁷, phenyl, COOR⁸ and halogen, with R⁵, R⁶, R⁷ and R⁸ independently represent hydrogen or alkyl C₁₋₆, and
iii) X represents an acidic moiety,
pharmaceutically acceptable salts thereof,
for the preparation of a medicament intended to be used in the prevention and/or the reduction of the occurring of an excess of cholesterol at peripheral tissues level, in an animal in need thereof.

**4.** The use according to anyone of claims 1 to 3, wherein said atherosclerosis and/or generation of atheromatous plaques and/or occurring of an excess of cholesterol at peripheral tissues level results in intermittent claudication, myocardial infarction, coronary artery disease, coronary heart disease, stable or unstable angina pectoris, stroke, and/or transient ischemic attack.

**5.** The use according to any one of claims 1 to 4, wherein R¹ and R² are an halogen, identical or different, chosen among Cl, F, Br and I, and in particular are identical, and more particularly are Cl.

**6.** The use according to any one of claims 1 to 5, wherein R³ is chosen among ethyl, butyl, methylethyl, methoxyethyl, methylthioethyl, trifluoroethyl, methoxycarbonylmethyl, dimethylaminoethyl, cyclopentyl, and phenyl, and in particular is methylthioethyl.

**7.** The use according to any one of claims 1 to 6, wherein R⁴ is chosen among propyl, trifluoropropyl and cyclohexyl, and in particular is trifluoropropyl.

**8.** The use according to any one of claims 1 to 7, wherein X is a phosphoric acid moiety.

**9.** The use according to any one of claims 1 to 8, wherein the compound of formula (I) is *N-*[2-(methylthio)ethyl]-2-[(3,3,3-trifluoropropyl)thio]-5'-adenylic acid, monoanhydride with dichloromethylenebisphosphonic acid, a pharmaceutically acceptable salt, a metabolite or a prodrug thereof.

**10.** The use according to any one of claims 1 to 9, wherein said compound of formula (I) is used at a therapeutically effective amount from about 0.1 mg/kg/day to about 1000 mg/kg/day, in particular from about 0.1 mg/kg/day to about 100 mg/kg/day, more particularly from about 0.1 mg/kg/day to about 50 mg/kg/day, and in particular from about 0.1 mg/kg/day to about 10 mg/kg/day.

**11.** The use according to any one of claims 1 to 10, wherein said compound of formula (I) is used in combination with a second compound allowing the increase of plasma level of HDL-cholesterol and/or the decrease of plasma level of LDL-cholesterol.

**12.** The use according to claim 11, wherein said second compound is chosen among statins, fibrates, thiazolidinediones and nicotinic acid derivatives.

**13.** The use according to any one of claims 1 to 12, wherein said animal is a human or a non-human mammal in need thereof.

**14.** The use according to claim 13, wherein said non-human mammal is chosen among domestic animals, laboratory animals or agricultural animals.

**15.** A pharmaceutical composition comprising, in combination, a compound of formula (I) as defined in any one of claims 1 to 3, 5 to 9, and a second compound allowing the increase of plasma level of HDL-cholesterol and/or decrease of plasma level of LDL-cholesterol.

**16.** The pharmaceutical composition according to claim 15, wherein said second compound is chosen among statins, fibrates, thiazolidinediones and nicotinic acid derivatives.

**17.** The pharmaceutical composition according to claim 15 or 16, wherein said compound of formula (I) and said second compound are presented separately or simultaneously in a same container.

**18.** The pharmaceutical composition according to any one of claims 15 to 17, for use in the prevention and/or the treatment of cardiovascular diseases related to atherosclerosis and/or generation of atheromatous plaques.

**19.** Method for screening a compound modulating HDL-cholesterol internalization comprising at least a step of exposing a sample of cells expressing a P2Y₁₃ receptor to a compound to be tested under conditions favourable for internalization of said HDL-cholesterol by said sample of cells and a step of detecting optional internalization.

**20.** A method according to claim 19 said method comprising the steps of:
a) incubating a first sample of said cells in the presence of said compound and HDL-cholesterol and a second sample of said cells in the absence of said compound and in presence of HDL-cholesterol, both said samples being under conditions which permit binding of said compound to P2Y₁₃ receptor and internalization of HDL-cholesterol,
b) detecting internalization of HDL-cholesterol in said first and second sample and,
c) comparing internalization of HDL-cholesterol into said first and second samples.

**21.** The method according to claim 20, wherein said HDL-cholesterol is chosen from triglycerides-rich HDL₂, cholesteryl-ether-labeled triglycerides-riche HDL₂, esters of HDL-cholesterol, HDL₃ and cholesteryl-ether-labeled HDL₃.

**22.** The method according to claims 20 or 21, wherein said first and second samples of cells are chosen among hepatic cell lines, hepatic cell of primary cultures, hepatic cells of explanted liver tissues, hepatic cells of *in vitro* isolated liver and hepatic cells of *in vivo* liver, and in particular is HepG₂ or HuH7 cell line.

**23.** The method according to any one of claims 19 to 22, wherein said cells of said sample of cells express a receptor binding HDL-cholesterol, said receptor is radiolabelled or fluorescent-labelled, using green-fluorescent protein (GFP) labelling, and in particular is chosen from SR-BI, SR-BII and CLA-1 receptor.
